# EUROPEAN PATENT APPLICATION

(11) **EP 4 740 958 A1**
(43) Date of publication of application: **13.05.2026**
(21) Application number: 24838692.2
(22) Date of filing: 05.07.2024
(51) Int. Cl.: A61K 39/395, A61P 35/00

(54) **PHARMACEUTICAL PREPARATION OF ANTI-HUMAN CCR8 MONOCLONAL ANTIBODY AND USE THEREOF**

(30) Priority: 07.07.2023 CN 202310827153
(71) Applicant: QILU PHARMACEUTICAL CO., LTD., Jinan, Shandong 250100 (CN); Shanghai Qilu Pharmaceutical Research and Development Centre Ltd., Shanghai 201203 (CN)
(72) Inventor: LI, Huihui, Jinan, Shandong 250100 (CN); ZHANG, Le, Jinan, Shandong 250100 (CN); AN, Zhenming, Jinan, Shandong 250100 (CN); LIU, Jun, Jinan, Shandong 250100 (CN); SUN, Lixia, Jinan, Shandong 250100 (CN); LI, Na, Jinan, Shandong 250100 (CN); SHEN, Qinyong, Jinan, Shandong 250100 (CN); YANG, Yong, Jinan, Shandong 250100 (CN); CAO, Yawen, Jinan, Shandong 250100 (CN)
(74) Representative: ABG Intellectual Property Law, S.L.
(86) International application number: PCT/CN2024/103735
(87) International publication number: WO 2025/011439

(57) **Abstract**

Provided are a stable pharmaceutical preparation of an anti-human CCR8 monoclonal antibody and the use thereof. The pharmaceutical preparation of the anti-human CCR8 antibody is very stable, can still meet the requirements for pharmaceutical use after enduring long-term storage, high temperatures or low temperatures, freeze-thaw cycles and oscillation, and has wide application prospects.

## Description

### TECHNICAL FIELD

The present disclosure belongs to the field of antibody preparations, and specifically relates to a pharmaceutical preparation of an anti-human CCR8 monoclonal antibody and use thereof.

### BACKGROUND

Immune checkpoint inhibitors, such as PD-1/PD-L1 drugs, have achieved good therapeutic effects in the treatment of various tumors, and however, their efficacy rates are still relatively low, ranging from about 15% to 40%, depending on different cancer types. In addition, some patients who initially showed clinical responses develop drug resistance over time, possibly due to compensatory mechanisms in tumor microenvironment (TME) to escape the anti-tumor effects of immune checkpoint inhibitors.

Tregs are one of CD4+ T cell lineages, and specifically express Forkhead box P3 (Foxp3). They can be divided into naive, effector and non-Treg cells according to their different sources. Naive Treg cells have a weak immunosuppressive function, and can proliferate and differentiate into effector Treg cells after stimulation by T cell receptors (TCRs). Effector Treg cells are in a terminally differentiated state of immune and have strong immunosuppressive effects, and mainly exert immunosuppression by releasing inhibitory cytokines, directly killing antigen-presenting cells, inhibiting immune function through immune checkpoints, and regulating metabolism such as adenosine pathways. Non-Treg cells have no immunosuppressive activity, but produce inflammatory cytokines. Generally speaking, the number of effector Treg cells is 1%-5% in human peripheral blood, and is about 10%-50% in the TME. Existing studies have shown that a high proportion of Treg infiltration in the TME is negatively correlated with the survival rate of different types of tumors, and therefore, activation of tumor immune responses by targeted clearance of Tregs is expected to be used to enhance tumor immunity.

Currently, classic Treg-related targets such as CD25, CTLA4, and CCR4 have been proven to inhibit tumor growth by eliminating Tregs, but these targets are also highly expressed on T effector cells, which can easily lead to adverse reactions. Therefore, there is an unmet clinical need to find targets that are specifically expressed on tumor Tregs and have low or no expression on T effector cells for further development.

C-C chemokine receptor 8 (CCR8) protein is one member of the chemokine receptor subfamily and belongs to the G protein-coupled receptor, and its ligands include CCL1 and so on. The main role of CCR8 is to participate in the recruitment of Tregs and Th2 cells to inflammatory and tumor sites. The expression of CCR8 target is highly specific. Studies have shown that CCR8 is expressed at low levels on Treg cells, CD4+T cells, and CD8+T cells in the peripheral blood of tumor patients, while its expression is highly upregulated on Treg cells in the corresponding tumor tissues of the patients, and its expression is low on CD4+ and CD8+T cells in tumor tissues. The expression of CCR8 in different tissues of normal mice and tumor model mice was compared in other literature. The results showed that CCR8 was very low or almost not expressed in normal mouse tissues, whereas was highly expressed in the tumor tissues of tumor-bearing mice, with low expression in spleen and lymph nodes, and almost no expression in other tissues. In summary, an antibody targeting CCR8 can specifically eliminate Tregs in tumor tissues and alleviate the immunosuppression of tumor tissues. It is expected that an antibody targeting CCR8 in combination with PD-1/PD-L1 drugs has a synergistic effect, and will not affect tumor-infiltrating T effector cells, resulting in a lower risk of systemic autoimmunity and better safety.

### SUMMARY OF THE INVENTION

In order to pursue a better clinical effect, an anti-human CCR8 monoclonal antibody, named J06 is firstly obtained in the present disclosure.

Based on the J06 antibody, further exploration and research had been conducted on the formulation of J06 preparation, and ultimately, a solution preparation that was most suitable for monoclonal antibody J06 and can stably preserve the monoclonal antibody was obtained. This preparation can fully prevent the aggregation, degradation, oxidation or denaturation or the like of the monoclonal antibody J06 protein, thereby maintaining the biological activity of its effective components, and is suitable for clinical use. Further, based on the monoclonal antibody J06 preparation, the pharmacological function of the preparation was deeply studied, and it was found that the preparation had a good anti-tumor activity.

### DETAILED DESCRIPTION OF DISCLOSURE

### 1. Terminology

All publications, patents, and patent applications mentioned in this specification are herein incorporated by reference to the same extent as if each individual publication, patent, or patent application was specifically and individually indicated to be incorporated by reference.

Before the present disclosure is described in detail below, it is to be understood that this disclosure is not limited to the particular methodology, protocols, and reagents described herein as these may vary. It is also to be understood that the terminology used herein is for the purpose of describing particular embodiments only, and is not intended to limit the scope of the present disclosure. Unless defined otherwise, all technical and scientific terms used herein have the same meaning as commonly understood by one of ordinary skill in the art to which this disclosure belongs.

Certain embodiments disclosed herein include numerical ranges, and certain aspects of the disclosure may be described by means of ranges. Unless otherwise specified, it should be understood that numerical ranges or descriptions by means of ranges are only for the purpose of simplicity and convenience, and should not be considered as a strict limitation on the scope of the present disclosure. Therefore, descriptions by means of ranges should be considered to have specifically disclosed all the possible sub-ranges as well as all the possible specific numerical points within that range, as if such sub-ranges and numerical points were explicitly recited herein. The above principles apply equally regardless of the width or narrowness of the values stated. When a range is described, the range includes the endpoints of the range.

When referring to a measurable value such as an amount, or a temporal duration, etc., the term "about" is meant to include variations of ±20%, or in some cases ±10%, or in some cases ±5%, or in some cases ±1%, or in some cases ±0.1% of the specified value.

The term "antibody" as used herein typically refers to a Y-shaped tetrameric protein comprising two heavy (H) polypeptide chains and two light (L) polypeptide chains held together by covalent disulfide bonds and non-covalent interactions. A natural IgG antibody has such a structure.
Each light chain comprises a light chain variable domain (VL) and a light chain constant domain (CL). Each heavy chain comprises a heavy chain variable domain (VH) and a heavy chain constant domain (CH) (or called a heavy chain constant region (CH)).

The term "monoclonal antibody" (or "mAb") refers to an antibody produced by a single cell clone that is essentially homogeneous and is directed against only a specific antigenic epitope. A monoclonal antibody can be prepared using a variety of techniques known in the art, including hybridoma technology, recombinant technology, phage display technology, transgenic animals, synthetic technology, or a combination of the above technologies.

### 2. Summary of the Invention

The present disclosure aims to provide a stable preparation that is suitable for an anti-human CCR8 monoclonal antibody and the use thereof.

The present disclosure provides a technical solution of a pharmaceutical preparation of an anti-human CCR8 monoclonal antibody, comprising an anti-human CCR8 monoclonal antibody or an antigen-binding fragment thereof and a buffer, wherein the sequences of the three CDRs in the heavy chain variable region of the anti-human CCR8 monoclonal antibody or antigen-binding fragment thereof are SEQ ID NO: 1, SEQ ID NO: 2, and SEQ ID NO: 3, respectively; and the sequences of the three CDRs in the light chain variable region of the anti-human CCR8 monoclonal antibody or antigen-binding fragment thereof are SEQ ID NO: 4, SEQ ID NO: 5, and SEQ ID NO: 6, respectively; and the buffer is a sodium acetate buffer, a histidine buffer, or a sodium citrate buffer.

Preferably, the sequence of the heavy chain variable region of the anti-human CCR8 monoclonal antibody or antigen-binding fragment thereof is set forth in SEQ ID NO: 7, and the sequence of the light chain variable region of the anti-human CCR8 monoclonal antibody or antigen-binding fragment thereof is set forth in SEQ ID NO: 8.

Preferably, the sequence of the heavy chain of the anti-human CCR8 monoclonal antibody or antigen-binding fragment thereof is set forth in SEQ ID NO: 9, and the sequence of the light chain of the anti-human CCR8 monoclonal antibody or antigen-binding fragment thereof is set forth in SEQ ID NO: 10. Preferably, the preparation further comprises a stabilizer selected from sucrose, trehalose or sorbitol.

Preferably, the preparation further comprises a solubilizer selected from polysorbate 20, polysorbate 80 or poloxamer 188.

Preferably, the pH value of the preparation is 4.5-6.0.

Preferably, the content of the anti-human CCR8 monoclonal antibody or antigen-binding fragment thereof in the preparation is 20-160 mg/ml, and preferably, its content is 20 mg/ml, 50 mg/ml, 80 mg/ml, 100 mg/ml, 120 mg/ml or 160 mg/ml.

Preferably, the content of sodium acetate in the preparation is 5-20 mmol/L, and preferably, its content is 5 mmol/L, 10 mmol/L, 15 mmol/L or 20 mmol/L.

Preferably, the content of sucrose in the preparation is 50-90 mg/ml, and preferably, its content is 50 mg/ml, 70 mg/ml, 80 mg/ml or 90 mg/ml.

Preferably, the content of polysorbate 80 in the preparation is 0.04-2 mg/ml, and preferably, its content is 0.04 mg/ml, 0.2 mg/ml, 0.5 mg/ml, 0.7 mg/ml or 2 mg/ml.

Preferably, the content of polysorbate 20 in the preparation is 0.5-2 mg/ml, and preferably, its content is 0.5 mg/ml, 0.7 mg/ml or 2 mg/ml.

Preferably, the preparation comprises 20-160 mg/ml of the anti-human CCR8 monoclonal antibody or antigen-binding fragment thereof, 5-20 mmol/L of sodium acetate, 50-90 mg/ml of sucrose, and 0.5-2 mg/ml of polysorbate 20, and the pH value of the preparation is 4.5-6.0.

Preferably, the preparation comprises 20-160 mg/ml of the anti-human CCR8 monoclonal antibody or antigen-binding fragment thereof, 5-20 mmol/L of sodium acetate, 50-90 mg/ml of sucrose, and 0.04-2 mg/ml of polysorbate 80, and the pH value of the preparation is 4.5-6.0.

Preferably, the preparation comprises 20-30 mg/ml of the anti-human CCR8 monoclonal antibody or antigen-binding fragment thereof, 10 mmol/L of sodium acetate, 90 mg/ml of sucrose, and 0.5 mg/ml of polysorbate 20, and the pH value of the preparation is 4.5-6.0.

Preferably, the preparation comprises 20-30 mg/ml of the anti-human CCR8 monoclonal antibody or antigen-binding fragment thereof, 10 mmol/L of sodium acetate, 90 mg/ml of sucrose, and 0.5 mg/ml of polysorbate 80, and the pH value of the preparation is 4.5-6.0.

Another technical solution provided by the present disclosure is a lyophilized preparation, which is obtained by freeze-drying the above pharmaceutical preparation.

Another technical solution provided by the present disclosure is use of the above pharmaceutical preparation or the above lyophilized preparation in the manufacture of a medicament for treating advanced solid tumors.

The preparation of the present disclosure has the following beneficial effects.

The preparation of the present disclosure is very stable, can still meet the requirements for pharmaceutical use after enduring long-term storage, high temperatures or low temperatures, freeze-thaw cycles and oscillation, and has wide application prospects.

### BRIEF DESCRIPTION OF THE DRAWINGS

Figure 1 is a graph of monomer purity determined by SE-HPLC in pH range screening tests.
Figure 2 is a graph of the main peak purity determined by nrCE-SDS in pH range screening tests.

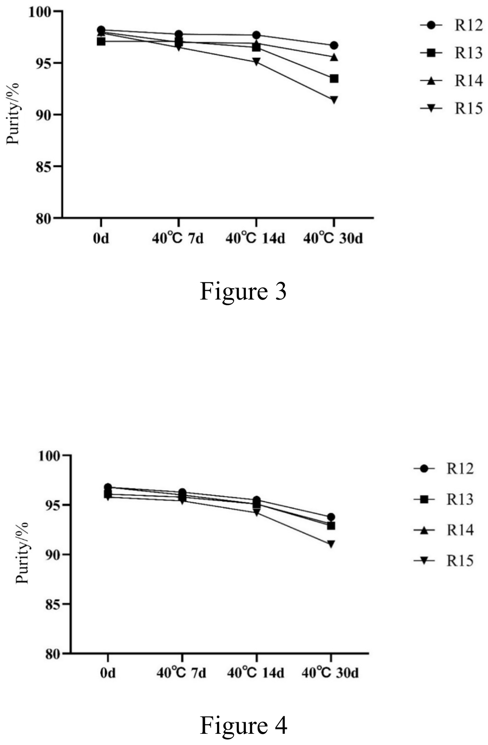

Figure 3 is a graph of monomer purity determined by SE-HPLC in excipient screening tests.
Figure 4 is a graph of the main peak purity determined by nrCE-SDS in excipient screening tests.

### DETAILED DESCRIPTION

The present disclosure is further illustrated in detail by the following examples. It is still regarded as a part of the present disclosure to change the concentration of the components in the preparation or add other substances into the preparation without significantly affecting the stability of the anti-human CCR8 antibody based on the present disclosure.

### Size Exclusion Chromatography (SE-HPLC)

Size exclusion chromatography was used to determine the contents of high molecular weight substances (HMWS), monomers and low molecular weight substances (LMWS) in this product. The method was performed using an X Bridge BEH 200 Å SEC chromatographic column (Waters Corporation) on a Waters e2695-2489 HPLC system (Waters Corporation). The detection wavelength was 215 nm; the mobile phase was 100 mM sodium phosphate, and 250 mM sodium chloride buffer, pH 7.3; the flow rate was 0.5 ml/min, and the run time was 30 min. The test sample was diluted with the mobile phase to a protein content of 1 mg/ml, and 20 µg was injected. Integration processing was performed using Empower 3 software (Waters Corporation).

### Capillary Electrophoresis (CE-SDS)

The main peak and (LC+HC) purities were determined by non-reducing CE-SDS (nrCE) and reducing CE-SDS (rCE), respectively. This assay was performed on a SCIEX PA800 plus capillary electrophoresis system using a 50 µm I.D. non-coated quartz capillary with an effective separation length of 20 cm (total length: 30.2 cm) and a PDA at 220 nm with a bandwidth of 10 nm.

### Surface Plasmon Resonance Technology (SPR)

Surface Plasmon Resonance technology (SPR) was used to determine the affinity of this product for a recombinant human CCR8. Direct immobilization method was used in this method for analysis, and the recombinant human CCR8 protein was firstly fixed on the experimental channel of the CM5 chip by amino coupling, with a coupling amount of approximately 15RU. 1 nM of this product was serially diluted to 31.25pM with the running buffer, and the products with a serial concentration range of 1nM-31.25pM were successively flowed through the reference channel and the experimental channel, respectively, with association for 3 minutes and dissociation for 10 minutes; after each cycle was completed, regeneration was performed twice with 50 mM HCl for 30 seconds each. The obtained data were analyzed using Biacore 8K Evaluation software and fitted using a 1:1 binding model.

The following specific examples are set forth to illustrate the present disclosure, and it should be understood that these examples are only for illustrating the present disclosure, rather than limiting the scope of the present disclosure.

### Example 1: Attainment and Sequence Information of Anti-human CCR8 Monoclonal Antibody J06

According to the contents described in PCT/US2023/060686, the anti-human CCR8 monoclonal antibody J06 (corresponding to the anti-CCR8 Mab 1187 in the above patent application) was obtained. The sequences of the three CDRs in the heavy chain variable region of the anti-human CCR8 monoclonal antibody J06 were SEQ ID NO: 1, SEQ ID NO: 2 and SEQ ID NO: 3, respectively, and the sequences of the three CDRs in the light chain variable region of the anti-human CCR8 monoclonal antibody J06 were SEQ ID NO: 4, SEQ ID NO: 5 and SEQ ID NO: 6, respectively. The sequence of the heavy chain variable region of the anti-human CCR8 monoclonal antibody J06 was set forth in SEQ ID NO: 7, and the sequence of the light chain variable region of the anti-human CCR8 monoclonal antibody J06 was set forth in SEQ ID NO: 8. The sequence of the heavy chain of the anti-human CCR8 monoclonal antibody J06 was set forth in SEQ ID NO:9, and the sequence of the light chain of the anti-human CCR8 monoclonal antibody J06 was set forth in SEQ ID NO:10.

### Example 2: Preparation of Anti-human CCR8 Monoclonal Antibody J06

The anti-human CCR8 monoclonal antibody J06 was a monoclonal antibody expressed by CHO cells. The whole molecule of this monoclonal antibody consisted of two heavy chains (HCs) and two light chains (LCs), and each heavy chain consisted of 453 amino acid residues, and each light chain consisted of 219 amino acid residues. The above anti-human CCR8 monoclonal antibody J06 was expressed in CHO cells and purified to obtain a stock solution for further experiments.

### Example 3: Solubilizer Screening Tests

The protein used in the following experiments was the anti-human CCR8 monoclonal antibody J06 prepared in Example 2. Based on the formulation comprising 30 mg/ml of the protein, 90 mg/ml of sucrose, and the buffer system of 10 mmol/L of sodium acetate, the types (polysorbate 20, and polysorbate 80) and amounts of solubilizers were screened. The formulation design was shown in Table 1.

**Table 1. Formulations used in solubilizer screening tests**

| Formulation No. | Protein concentration | Solubilizer concentration | Solubilizer type | Stabilizer | Buffer system | pH value |
|---|---|---|---|---|---|---|
| R0 | 30mg/ml | / | / | 90mg/ml sucrose | 10mmol/L sodium acetate | 5.0 |
| R1 | | 0.2mg/ml | Polysorbate 20 | | | |
| R2 | | 0.5mg/ml | | | | |
| R3 | | 0.2mg/ml | Polysorbate 80 | | | |
| R4 | | 0.5mg/ml | | | | |

The samples prepared according to Table 1 were subjected to influencing factor tests: the samples were taken for testing on day 0, week 1, week 2, and week 3, respectively, under high temperature conditions (40°C±2°C); the samples were taken for testing at 24 and 48 hours, respectively, under oscillation conditions; the samples were taken for testing at 6 and 10 times under freezing-thawing conditions (freezing at -80°C for 16 hours and thawing at 25°C for 8 hours as one cycle).

Inspection objects: appearance, presence or absence of visible foreign matters, protein content, size exclusion chromatography (SE-HPLC) purity, and non-reducing capillary electrophoresis (nrCE-SDS) purity.

The test results were shown in Table 2.

**Table 2. Results of solubilizer screening tests**

| Sample | Investigation condition | Appearance | Presence or absence of visible foreign matters | Protein content (mg/ml) | SE-HPLC (%) monomer | nrCE-SDS (%) main peak | ADCC activity (%) |
|---|---|---|---|---|---|---|---|
| R0 | | Colorless to light yellow, and clear to slightly opalescent | Absence of visible foreign matters | 31.4 | 98.7 | 96.6 | 84 |
| R1 | | Colorless to light yellow, and clear to slightly opalescent | Absence of visible foreign matters | 30.6 | 98.7 | 96.7 | NA |
| R2 | 0d | Colorless to light yellow, and clear to slightly opalescent | Absence of visible foreign matters | 30.8 | 98.7 | 96.6 | NA |
| R3 | | Colorless to light yellow, and clear to slightly opalescent | Absence of visible foreign matters | 30.9 | 98.7 | 96.9 | NA |
| R4 | | Colorless to light yellow, and clear to slightly opalescent | Absence of visible foreign matters | 30.6 | 98.7 | 96.8 | NA |
| R0 | High temperature for 1W | Colorless to light yellow, and clear to slightly opalescent | Absence of visible foreign matters | 31.5 | 98.0 | 96.3 | NA |
| R1 | | Colorless to light yellow, and clear to slightly opalescent | Absence of visible foreign matters | 31.1 | 98.1 | 96.3 | NA |
| R2 | | Colorless to light yellow, and clear to slightly opalescent | Absence of visible foreign matters | 31.0 | 97.9 | 96.5 | NA |
| R3 | | Colorless to light yellow, and clear to slightly opalescent | Absence of visible foreign matters | 31.0 | 98.0 | 96.2 | NA |
| R4 | | Colorless to light yellow, and clear to slightly opalescent | Absence of visible foreign matters | 30.7 | 97.9 | 96.1 | NA |
| R0 | High temperature for 2W | Colorless to light yellow, and clear to slightly opalescent | Protein flocculation | 31.3 | 97.8 | 95.9 | NA |
| R1 | | Colorless to light yellow, and clear to slightly opalescent | Absence of visible foreign matters | 31.1 | 97.8 | 95.8 | NA |
| R2 | | Colorless to light yellow, and clear to slightly opalescent | Absence of visible foreign matters | 31.0 | 97.8 | 95.7 | NA |
| R3 | | Colorless to light yellow, and clear to slightly opalescent | Absence of visible foreign matters | 31.0 | 97.8 | 95.8 | NA |
| R4 | | Colorless to light yellow, and clear to slightly opalescent | Absence of visible foreign matters | 30.9 | 97.7 | 95.8 | NA |
| R0 | High temperature for 3W | Colorless to light yellow, and clear to slightly opalescent | Protein flocculation | 31.1 | 97.2 | 95.3 | NA |
| R1 | | Colorless to light yellow, and clear to slightly opalescent | Protein flocculation | 30.5 | 97.3 | 95.2 | 100 |
| R2 | | Colorless to light yellow, and clear to slightly opalescent | Absence of visible foreign matters | 30.7 | 97.2 | 95.3 | 114 |
| R3 | | Colorless to light yellow, and clear to slightly opalescent | Absence of visible foreign matters | 30.9 | 97.2 | 95.1 | 108 |
| R4 | | Colorless to light yellow, and clear to slightly opalescent | Absence of visible foreign matters | 30.9 | 97.2 | 95.1 | 124 |
| R0 | Oscillation for 24 hours | Colorless to light yellow, and clear to slightly opalescent | Protein flocculation | 31.5 | 98.6 | 96.9 | NA |
| R1 | | Colorless to light yellow, and clear to slightly opalescent | Absence of visible foreign matters | 30.9 | 98.7 | 96.7 | NA |
| R2 | | Colorless to light yellow, and clear to slightly opalescent | Absence of visible foreign matters | 30.9 | 98.7 | 97.0 | NA |
| R3 | | Colorless to light yellow, and clear to slightly opalescent | Absence of visible foreign matters | 30.7 | 98.5 | 96.8 | NA |
| R4 | | Colorless to light yellow, and clear to slightly opalescent | Absence of visible foreign matters | 30.6 | 98.6 | 96.5 | NA |
| R0 | Oscillation for 48 hours | Colorless to light yellow, and clear to slightly opalescent | Protein flocculation | 31.3 | 98.5 | 97.0 | 89 |
| R1 | | Colorless to light yellow, and clear to slightly opalescent | Absence of visible foreign matters | 31 | 98.7 | 96.8 | 108 |
| R2 | | Colorless to light yellow, and clear to slightly opalescent | Absence of visible foreign matters | 31.6 | 98.7 | 96.6 | 88 |
| R3 | | Colorless to light yellow, and clear to slightly opalescent | Absence of visible foreign matters | 31.1 | 98.7 | 96.8 | 90 |
| R4 | | Colorless to light yellow, and clear to slightly opalescent | Absence of visible foreign matters | 31 | 98.7 | 96.9 | 85 |
| R0 | 6 times of freezing-thawing | Colorless to light yellow, and clear to slightly opalescent | Absence of visible foreign matters | 31.3 | 98.6 | 96.7 | NA |
| R1 | | Colorless to light yellow, and clear to slightly opalescent | Absence of visible foreign matters | 30.5 | 98.6 | 96.8 | NA |
| R2 | | Colorless to light yellow, and clear to slightly opalescent | Absence of visible foreign matters | 30.7 | 98.7 | 96.8 | NA |
| R3 | | Colorless to light yellow, and clear to slightly opalescent | Absence of visible foreign matters | 31 | 98.6 | 96.5 | NA |
| R4 | | Colorless to light yellow, and clear to slightly opalescent | Absence of visible foreign matters | 31.3 | 98.5 | 96.9 | NA |
| R0 | 10 times of freezing-thawing | Colorless to light yellow, and clear to slightly opalescent | Protein flocculation | 31.4 | 98.6 | 96.9 | NA |
| R1 | | Colorless to light yellow, and clear to slightly opalescent | Absence of visible foreign matters | 30.9 | 98.8 | 96.9 | 111 |
| R2 | | Colorless to light yellow, and clear to slightly opalescent | Absence of visible foreign matters | 30.9 | 98.6 | 96.9 | 108 |
| R3 | | Colorless to light yellow, and clear to slightly opalescent | Absence of visible foreign matters | 30.9 | 98.7 | 96.9 | 107 |
| R4 | | Colorless to light yellow, and clear to slightly opalescent | Absence of visible foreign matters | 30.4 | 98.6 | 96.8 | 123 |

| | | | | | | | |
|---|---|---|---|---|---|---|---|
| Note: NA means that the experiment was not performed. | | | | | | | |

The test results showed that after being placed under high temperature (40°C±2°C) conditions for 3 weeks, the sample without solubilizer (R0) and the sample with 0.2 mg/ml polysorbate 20 (R1) had visible foreign matters of protein flocculation; the main peak purities of the samples (R1-R4) comprising different types (polysorbate 20 or polysorbate 80) and concentrations (0.2 mg/ml or 0.5 mg/ml) of solubilizers showed downward trends, which were basically the same. After 48 hours of oscillation and 10 times of freezing-thawing , there was no significant change in each of the detection indicators for the formulations R1-R4. Based on the test results, both 0.5 mg/ml of polysorbate 20 and 0.2 mg/ml or 0.5 mg/ml of polysorbate 80 can be used as solubilizers for J06.

### Example 4: pH Value Range Screening Tests

The pH value range was screened based on the formulation comprising 20 mg/ml of the protein, 90 mg/ml of sucrose, 0.5 mg/ml of polysorbate 80, and the buffer system of 10 mmol/L of sodium acetate. The pH value range was designed to be 4.0-6.0. The formulations were shown in Table 3.

**Table 3. Formulations used in pH value screening tests**

| Formulation No. | Protein concentration | Solubilizer | Stabilizer | Buffer system | pH |
|---|---|---|---|---|---|
| R5 | 20mg/ml | 0.5mg/ml of polysorbate 80 | 90mg/ml of sucrose | 10mmol/L of sodium acetate | 4.0 |
| R6 | | | | | 4.5 |
| R7 | | | | | 4.7 |
| R8 | | | | | 5.0 |
| R9 | | | | | 5.3 |
| R10 | | | | | 5.5 |
| R11 | | | | | 6.0 |

The formulations were prepared according to Table 3, and were sterilized by filtering, and then aliquoted. The prepared samples were subjected to influencing factor tests (high temperature at 40°C±2°C), and the samples were taken for testing on day 0, week 1, and week 2, respectively.

Inspection objects: appearance, protein content, presence or absence of visible foreign matters, size exclusion chromatography (SE-HPLC) purity, and non-reducing capillary electrophoresis (nrCE-SDS) purity. The test results were shown in Table 4.

**Table 4. Results of pH value screening tests**

| Sample | Investigation condition | Appearance | Presence or absence of visible foreign matters | Protein content (mg/ml) | SE-HPLC (%) monomer | nrCE-SDS (%) main peak |
|---|---|---|---|---|---|---|
| R5 | 0d | Colorless to light yellow, and clear to slightly opalescent | Absence of visible foreign matters | 22.0 | 98.2 | 96.4 |
| R6 | | Colorless to light yellow, and clear to slightly opalescent | Absence of visible foreign matters | 22.8 | 98.2 | 96.4 |
| R7 | | Colorless to light yellow, and clear to slightly opalescent | Absence of visible foreign matters | 22.7 | 98.1 | 96.4 |
| R8 | | Colorless to light yellow, and clear to slightly opalescent | Absence of visible foreign matters | 22.8 | 98.2 | 96.4 |
| R9 | | Colorless to light yellow, and clear to slightly opalescent | Absence of visible foreign matters | 22.7 | 98.2 | 96.4 |
| R10 | | Colorless to light yellow, and clear to slightly opalescent | Absence of visible foreign matters | 22.1 | 98.2 | 96.4 |
| R11 | | Colorless to light yellow, and clear to slightly opalescent | Absence of visible foreign matters | 21.4 | 98.2 | 96.4 |
| R5 | 40°C for 1W | Colorless to light yellow, and clear to slightly opalescent | Absence of visible foreign matters | 21.3 | 95.0 | NA |
| R6 | | Colorless to light yellow, and clear to slightly opalescent | Absence of visible foreign matters | 22.3 | 96.8 | NA |
| R7 | | Colorless to light yellow, and clear to slightly opalescent | Absence of visible foreign matters | 22.7 | 97.0 | NA |
| R8 | | Colorless to light yellow, and clear to slightly opalescent | Absence of visible foreign matters | 22.8 | 97.3 | NA |
| R9 | | Colorless to light yellow, and clear to slightly opalescent | Absence of visible foreign matters | 22.7 | 97.3 | NA |
| R10 | | Colorless to light yellow, and clear to slightly opalescent | Absence of visible foreign matters | 22.1 | 97.2 | NA |
| R11 | | Colorless to light yellow, and clear to slightly opalescent | Absence of visible foreign matters | 21.4 | 97.0 | NA |
| R5 | 40°C for 2W | Colorless to light yellow, and clear to slightly opalescent | Absence of visible foreign matters | 21.5 | 93.9 | 93.7 |
| R6 | | Colorless to light yellow, and clear to slightly opalescent | Absence of visible foreign matters | 22.6 | 96.1 | 94.3 |
| R7 | | Colorless to light yellow, and clear to slightly opalescent | Absence of visible foreign matters | 23.0 | 96.5 | 94.3 |
| R8 | | Colorless to light yellow, and clear to slightly opalescent | Absence of visible foreign matters | 23.1 | 96.8 | 94.8 |
| R9 | | Colorless to light yellow, and clear to slightly opalescent | Absence of visible foreign matters | 23.1 | 96.8 | 95.0 |
| R10 | | Colorless to light yellow, and clear to slightly opalescent | Absence of visible foreign matters | 22.4 | 96.8 | 94.9 |
| R11 | | Colorless to light yellow, and clear to slightly opalescent | Absence of visible foreign matters | 21.6 | 96.3 | 95.1 |

| | | | | | | |
|---|---|---|---|---|---|---|
| Note: NA means that the experiment was not performed. | | | | | | |

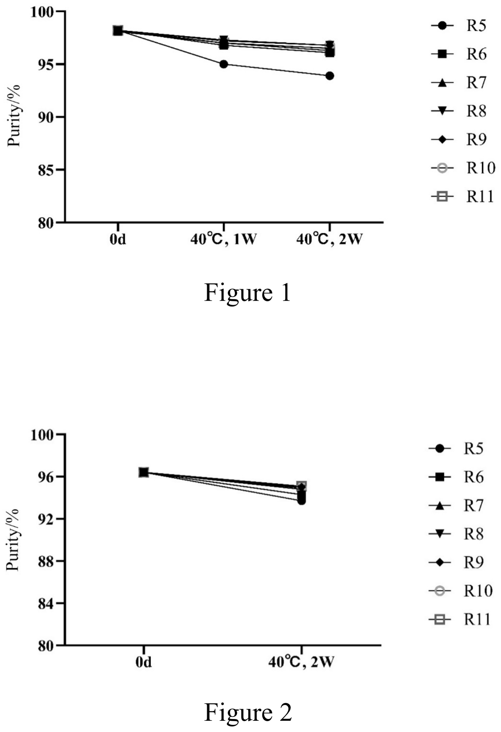

The test results showed that after the samples were under high temperature (40°C±2°C) condition for 2 weeks, there was no obvious change in the appearance, presence or absence of visible foreign matters, and protein content of each formulation. As shown in Figures 1 and 2, the SE-HPLC and nrCE-SDS purities of the sample (R1) with a pH value of 4.0 respectively decreased by 4.3% and 2.7% compared with the sample at time zero, with a significant decrease; each of the detection indicators for purities of the samples of formulations R6-R11 (pH 4.5-6.0) decreased slightly, but the downward trends were basically the same, and there was no significant difference among the formulations. Based on the test results of each formulation, the candidate pH value range was determined to be 4.5-6.0.

### Example 5: Excipient Screening Tests

The protein used in the following experiments was the anti-human CCR8 monoclonal antibody J06 prepared in Example 2. A variety of excipients were used for formulation screening, and the formulation design was shown in Table 5.

**Table 5. Formulations used in excipient screening tests**

| Formulation No. | Protein concentration | Buffer system | | Stabilizer | | Solubilizer | |
|---|---|---|---|---|---|---|---|
| | | Type | Concentration | Type | Concentration | Type | Concentration |
| R12 | 20 mg/ml | Sodium acetate | 10 mmol/L | Sucrose | 90 mg/ml | Polysorbate 80 | 0.04 mg/ml |
| R13 | 80 mg/ml | Histidine | 5 mmol/L | Trehalose | 100 mg/ml | Polysorbate 80 | 2 mg/ml |
| R14 | 20 mg/ml | Sodium citrate | 10 mmol/L | Sorbitol | 50 mg/ml | Polysorbate 80 | 0.5 mg/ml |
| R15 | 160 mg/ml | Histidine | 20 mmol/L | Sucrose | 50 mg/ml | Poloxamer 188 | 0.5 mg/ml |

The samples prepared according to Table 5 were subjected to influencing factor tests (high temperature at 40°C±2°C), and the samples were taken for testing on day 0, day 7, day 14, and day 30, respectively.

Inspection objects: appearance, presence or absence of visible foreign matters, protein content, size exclusion chromatography (SE-HPLC) purity, and non-reducing capillary electrophoresis (nrCE-SDS) purity. The test results were shown in Table 6.

**Table 6. Results of excipient screening tests**

| Sample | Investigation condition | Appearance | Presence or absence of visible foreign matters | Protein content (mg/ml) | SE-HPLC (%) monomer | nrCE-SDS (%) main peak | ADCC activity (%) |
|---|---|---|---|---|---|---|---|
| R12 | 0d | Colorless to light yellow, and clear to slightly opalescent | Absence of visible foreign matters | 22.2 | 98.2 | 96.8 | 90 |
| R13 | | Colorless to light yellow, and clear to slightly opalescent | Absence of visible foreign matters | 81.9 | 97.1 | 96.1 | 105 |
| R14 | | Colorless to light yellow, and clear to slightly opalescent | Absence of visible foreign matters | 20.6 | 98.0 | 96.8 | 119 |
| R15 | | Colorless to light yellow, and clear to slightly opalescent | Absence of visible foreign matters | 156.4 | 97.9 | 95.8 | 95 |
| R12 | 40°C for 7d | Colorless to light yellow, and clear to slightly opalescent | Absence of visible foreign matters | 22.3 | 97.8 | 96.3 | 106 |
| R13 | | Colorless to light yellow, and clear to slightly opalescent | Absence of visible foreign matters | 81.2 | 97.1 | 95.8 | 100 |
| R14 | | Colorless to light yellow, and clear to slightly opalescent | Absence of visible foreign matters | 20.4 | 97.0 | 96.0 | 106 |
| R15 | | Colorless to light yellow, and clear to slightly opalescent | Absence of visible foreign matters | 156.6 | 96.5 | 95.4 | 100 |
| R12 | 40°C for 14d | Colorless to light yellow, and clear to slightly opalescent | Absence of visible foreign matters | 22.4 | 97.7 | 95.5 | 104 |
| R13 | | Colorless to light yellow, and clear to slightly opalescent | Absence of visible foreign matters | 82.4 | 96.5 | 95.1 | 93 |
| R14 | | Colorless to light yellow, and clear to slightly opalescent | Absence of visible foreign matters | 20.5 | 96.9 | 95.1 | 98 |
| R15 | | Colorless to light yellow, and clear to slightly opalescent | Absence of visible foreign matters | 158.5 | 95.1 | 94.2 | 98 |
| R12 | 40°C for 30d | Colorless to light yellow, and clear to slightly opalescent | Absence of visible foreign matters | 22.4 | 96.7 | 93.8 | 110 |
| R13 | | Colorless to light yellow, and clear to slightly opalescent | Absence of visible foreign matters | 81.9 | 93.5 | 92.9 | 90 |
| R14 | | Colorless to light yellow, and clear to slightly opalescent | Absence of visible foreign matters | 20.5 | 95.6 | 93.1 | 83 |
| R15 | | Colorless to light yellow, and clear to slightly opalescent | Absence of visible foreign matters | 158.9 | 91.4 | 91.0 | 74 |

The detection results showed that after the samples were under high temperature (40°C±2°C) condition for 30 days, there was no obvious change in the appearance, presence or absence of visible foreign matters, and protein content of each formulation compared with the same at time zero, and each of the detection indicators for purities showed slight downward trends, which were basically the same; there was no significant difference in the various test indicators of each formulation under high temperature (40°C±2°C) condition for 30 days. Based on each of the detection results, the protein concentration can be selected from 20 to 160 mg/ml; sodium acetate, histidine, or sodium citrate can be used as a buffer; sucrose, trehalose, or sorbitol can be used as a stabilizer, and polysorbate 80 or poloxamer 188 can be used as a solubilizer.

Conclusion: Through the above formulation screening tests of the preparations, the buffer system can be selected from sodium acetate, histidine, or sodium citrate buffer system; the stabilizer can be selected from sucrose, trehalose, or sorbitol, and the solubilizer can be selected from polysorbate 20, polysorbate 80, or poloxamer 188.

### Example 6: Preparation Stability Test

Based on the above test results, the following preferred formulation was selected for stability study: 20 mg/ml of the protein, 90 mg/ml of sucrose, which served as a stabilizer; 0.5 mg/ml of polysorbate 80, which served as a solubilizer; 10 mmol/L of sodium acetate buffer, which served as a buffer system, pH value ranging from 4.5 to 6.0. A forced condition test (high temperature test), an acceleration test, and a long-term test were included. The samples were placed upright in the stability study; and the samples in the long-term test, the acceleration test, and the high temperature test were all packaged in imitation of the market. Each of the investigation conditions were shown in Table 7.

**Table 7. Investigation conditions of each stability test of preparation**

| Stability test | Investigation condition |
|---|---|
| Long-term test | 2~8°C |
| Acceleration test | 25°C±2°C, relative humidity of 60%±10% |
| High temperature test | 40°C±2°C |

The stability results under each of the above investigation conditions were shown in Tables 8-10.

**Table 8. Detection results of acceleration test**

| Investigation condition | Acceleration test | | | | |
|---|---|---|---|---|---|
| Investigation object | Criterion | Time (month) | | | |
| | | 0 | 1 | 3 | 6 |
| Protein content (mg/ml) | It should be 18.0~22.0mg/ml | 21.0 | 21.0 | 20.8 | 21.0 |
| ADCC activity (%) | It should be 50%~150% of the reference sample | 109 | 117 | 97 | 119 |
| SE-HPLC purity (%) | Monomer should be ≥95.0% | Monomer: 98.6 | Monomer: 98.3 | Monomer: 97.7 | Monomer: 96.9 |
| nrCE-SDS purity (%) | Main peak should be≥93.0% | Main peak: 97.0 | Main peak: 96.5 | Main peak: 95.6 | Main peak: 94.6 |

**Table 9. Detection results of high temperature test**

| Investigation condition | High temperature test | | | | |
|---|---|---|---|---|---|
| Investigation item | Criterion | Time (day) | | | |
| | | 0 | 10 | 20 | 30 |
| Protein content (mg/ml) | It should be 18.0~22.0mg/ml | 21.0 | 21.2 | 21.0 | 21.0 |
| ADCC activity (%) | It should be 50%~150% of the reference sample | 109 | 112 | 109 | 115 |
| SE-HPLC purity (%) | Monomer should be≥95.0% | Monomer: 98.6 | Monomer: 97.7 | Monomer: 96.6 | Monomer: 95.6 |
| nrCE-SDS purity (%) | Main peak should be≥93.0% | Main peak: 97.0 | Main peak: 96.0 | Main peak: 94.9 | Main peak: 93.6 |

**Table 10. Detection results of long-term test**

| Investigation condition | Long-term test | | | |
|---|---|---|---|---|
| Investigation item | Criterion | Time (month) | | |
| | | 0 | 3 | 6 |
| Protein content (mg/ml) | It should be 18.0~22.0mg/ml | 21.0 | 21.1 | 21.2 |
| ADCC activity (%) | It should be 50%~150% of the reference sample | 109 | 114 | 102 |
| SE-HPLC purity (%) | Monomer should be ≥95.0% | Monomer: 98.6 | Monomer: 98.5 | Monomer: 98.6 |
| nrCE-SDS purity (%) | Main peak should be ≥93.0% | Main peak: 97.0 | Main peak: 96.7 | Main peak: 96.8 |

The above results demonstrated that the antibody preparation has good stability and can still meet the quality standards after being investigated at 40°C±2°C in the high temperature test and at 25°C±2°C in the acceleration test.

### Example 7: Affinity Analysis of Anti-human CCR8 Monoclonal Antibody J06 Preparation and Human CCR8 Recombinant Protein

Based on the above results, affinity analysis was performed on the preferred preparation in Example 6. The affinity of the anti-human CCR8 monoclonal antibody J06 preparation for a recombinant human CCR8 protein was determined by SPR technology. The results were shown in Table 11, and the K_{D} value fitted by the affinity analysis of the J06 preparation for the recombinant human CCR8 was 7.77 pM. It can be seen that the J06 preparation had a high affinity for the recombinant human CCR8.

**Table 11. Kinetic fitting results of affinity analysis of J06 preparation for recombinant human CCR8**

| ka (1/Ms) | kd (1/s) | K_{D} (pM) | Rmax (RU) |
|---|---|---|---|
| 1.69E+07 | 1.31E-04 | 7.77E-12 | 16 |

## Claims

1. A pharmaceutical preparation of an anti-human CCR8 monoclonal antibody, comprising an anti-human CCR8 monoclonal antibody or an antigen-binding fragment thereof and a buffer, wherein the sequences of the three CDRs in the heavy chain variable region of the anti-human CCR8 monoclonal antibody or antigen-binding fragment thereof are SEQ ID NO: 1, SEQ ID NO: 2, and SEQ ID NO: 3, respectively; and the sequences of the three CDRs in the light chain variable region of the anti-human CCR8 monoclonal antibody or antigen-binding fragment thereof are SEQ ID NO: 4, SEQ ID NO: 5, and SEQ ID NO: 6, respectively; and the buffer is a sodium acetate buffer, a histidine buffer, or a sodium citrate buffer.

2. The pharmaceutical preparation according to claim 1, wherein the sequence of the heavy chain variable region of the anti-human CCR8 monoclonal antibody or antigen-binding fragment thereof is set forth in SEQ ID NO: 7, and the sequence of the light chain variable region of the anti-human CCR8 monoclonal antibody or antigen-binding fragment thereof is set forth in SEQ ID NO: 8.

3. The pharmaceutical preparation according to claim 2, wherein the sequence of the heavy chain of the anti-human CCR8 monoclonal antibody or antigen-binding fragment thereof is set forth in SEQ ID NO: 9, and the sequence of the light chain of the anti-human CCR8 monoclonal antibody or antigen-binding fragment thereof is set forth in SEQ ID NO: 10.

4. The pharmaceutical preparation according to claim 1, wherein the preparation further comprises a stabilizer selected from sucrose, trehalose or sorbitol.

5. The pharmaceutical preparation according to claim 1, wherein the preparation further comprises a solubilizer selected from polysorbate 20, polysorbate 80 or poloxamer 188.

6. The pharmaceutical preparation according to claim 1, wherein the pH value of the preparation is 4.5-6.0.

7. The pharmaceutical preparation according to claim 1, wherein the content of the anti-human CCR8 monoclonal antibody or antigen-binding fragment thereof in the preparation is 20-160 mg/ml, and preferably 20 mg/ml, 50 mg/ml, 80 mg/ml, 100 mg/ml, 120 mg/ml or 160 mg/ml.

8. The pharmaceutical preparation according to claim 1, wherein the content of sodium acetate in the preparation is 5-20 mmol/L, and preferably, 5 mmol/L, 10 mmol/L, 15 mmol/L or 20 mmol/L.

9. The pharmaceutical preparation according to claim 4, wherein the content of sucrose in the preparation is 50-90 mg/ml, and preferably, 50 mg/ml, 70 mg/ml, 80 mg/ml or 90 mg/ml.

10. The pharmaceutical preparation according to claim 5, wherein the content of polysorbate 80 in the preparation is 0.04-2 mg/ml, and preferably, 0.04 mg/ml, 0.2 mg/ml, 0.5 mg/ml, 0.7 mg/ml or 2 mg/ml.

11. The pharmaceutical preparation according to claim 5, wherein the content of polysorbate 20 in the preparation is 0.5-2 mg/ml, and preferably, 0.5 mg/ml, 0.7 mg/ml or 2 mg/ml.

12. The pharmaceutical preparation according to any one of claims 1-11, wherein the preparation comprises 20-160 mg/ml of the anti-human CCR8 monoclonal antibody or antigen-binding fragment thereof, 5-20 mmol/L of sodium acetate, 50-90 mg/ml of sucrose, and 0.5-2 mg/ml of polysorbate 20, and the pH value of the preparation is 4.5-6.0.

13. The pharmaceutical preparation according to any one of claims 1-11, wherein the preparation comprises 20-160 mg/ml of the anti-human CCR8 monoclonal antibody or antigen-binding fragment thereof, 5-20 mmol/L of sodium acetate, 50-90 mg/ml of sucrose, and 0.04-2 mg/ml of polysorbate 80, and the pH value of the preparation is 4.5-6.0.

14. The pharmaceutical preparation according to claim 12, wherein the preparation comprises 20-30 mg/ml of the anti-human CCR8 monoclonal antibody or antigen-binding fragment thereof, 10 mmol/L of sodium acetate, 90 mg/ml of sucrose, and 0.5 mg/ml of polysorbate 20, and the pH value of the preparation is 4.5-6.0.

15. The pharmaceutical preparation according to claim 13, wherein the preparation comprises 20-30 mg/ml of the anti-human CCR8 monoclonal antibody or antigen-binding fragment thereof, 10 mmol/L of sodium acetate, 90 mg/ml of sucrose, and 0.5 mg/ml of polysorbate 80, and the pH value of the preparation is 4.5-6.0.

16. A lyophilized preparation, which is obtained by freeze-drying the pharmaceutical preparation according to any one of claims 1-15.

17. Use of the pharmaceutical preparation according to any one of claims 1-15 or the lyophilized preparation according to claim 16 in the manufacture of a medicament for treating advanced solid tumors.
